# FASCICULE DE BREVET EUROPEEN

(11) **EP 1 124 799 B1**
(45) Date de publication et mention de la délivrance du brevet: **14.05.2003**
(21) Numéro de dépôt: 99950881.5
(22) Date de dépôt: 29.10.1999
(51) Int. Cl.: C07C 319/20, C07C 323/60, C07C 323/58, C12P 13/12

(54) **PROCEDE DE PREPARATION DE LA METHIONINE**
VERFAHREN ZUR HERSTELLUNG VON METHIONIN
METHOD FOR PREPARING METHIONINE

(30) Priorité: 06.11.1998 FR 9814000
(43) Date de publication de la demande: 22.08.2001
(73) Titulaire: AVENTIS ANIMAL NUTRITION S.A., 92164 Antony Cedex (FR)
(72) Inventeur: PONCEBLANC, Hervé, F-69100 Villeurbanne (FR); FAVRE-BULLE, Olivier, F-69003 Lyon (FR); GROS, Georges, F-92160 Antony (FR)
(74) Mandataire: Guerre, Dominique
(86) Numéro de dépôt international: FR9902650
(87) Numéro de publication internationale: WO00027809

(56) Documents cités:
- EP-A- 0 084 470
- EP-A- 0 168 282
- EP-A- 0 228 938
- US-A- 4 459 423
- US-A- 5 672 745

## Description

La présente invention concerne un procédé de préparation de l'aminoamide de la méthionine. Elle concerne aussi la préparation de la méthionine sans coproduction de sels, à partir d'une solution aqueuse contenant essentiellement l'aminonitrile de la méthionine (ou amino-2, méthylthio-4, butyronitrile, noté AMTBN) par production au cours d'une première étape de l'aminoamide suivi par deux étapes complémentaires.

Le procédé de préparation de l'aminoamide de la méthionine (ou amine-2, méthylthio-4, butyramide, noté AMTBM), consiste à hydrater l'aminonitrile de la méthionine en présence d'un catalyseur de type cétone et d'une résine basique de type OH. Cette hydratation permet l'obtention d'une solution contenant l'aminoamide de la méthionine complètement exempte de sels minéraux.

Le procédé de préparation de la méthionine consiste en un procédé en trois étapes dont la première est l'étape précédemment décrite:
- l'aminonitrile est hydraté en aminoamide de la méthionine en présence d'un catalyseur de type cétone et d'une résine basique de type OH,
- l'aminoamide de la méthionine est hydrolysé en méthioninate d'ammonium, par différentes voies possibles :
   * par voie chimique en catalyse homogène telle que par exemple l'hydrolyse à l'ammoniaque,
   * par voie chimique en catalyse hétérogène telle que par exemple une hydrolyse catalysée par des oxydes,
   * par voie enzymatique,
- enfin la méthionine est récupérée, à partir du méthioninate d'ammonium, après entraînement de l'ammoniac par stripping.

Il est connu, par exemple selon les brevets EP 84470 ou EP 168282, que l'hydratation de l'aminonitrile peut être réalisée sur résine polymère greffée avec des groupements de type cétonique ( -(C=O)-), en présence d'ions hydroxydes. La mise en oeuvre d'un tel procédé, utilisant une base alcaline forte (soude, potasse, ...) pour fournir les ions hydroxydes nécessaires à la catalyse, entraîne l'obtention d'un aminoamide pollué par l'alcalin correspondant.

D'autre part, il est connu, par exemple selon le brevet EP 228938, de préparer la méthionine à partir d'aminoamide de méthionine en utilisant une base forte (par exemple la soude ou autre base alcaline) pour réaliser l'hydrolyse alcaline de l'amide. La mise en oeuvre d'un tel procédé entraîne, lors de l'acidification du méthioninate alcalin obtenu par un acide minéral fort (sulfurique ou chlorhydrique), la coproduction d'un sel minéral : sulfate ou chlorure du métal alcalin correspondant.

Dans les 2 procédés cités ci-dessus, l'utilisation d'une base alcaline forte comme catalyseur d'hydratation de l'aminonitrile et/ou comme réactif d'hydrolyse de l'amide, entraîne la coproduction d'un sel minéral : sulfate ou chlorure du métal alcalin correspondant, qu'il faut ensuite séparer de la méthionine, avec souvent beaucoup de difficultés, selon des procédés coûteux de cristallisation, filtration, concentration successives des eaux-mères.

Le procédé selon l'invention permet d'éviter l'utilisation d'une base alcaline et donc la coexistence dans le même flux de la méthionine et du sel minéral, qu'il faut séparer ensuite comme décrit ci-dessus.

Ainsi, le procédé selon l'invention permet de produire un flux de méthionine exempt de sel minéral.

L'aminonitrile de la méthionine (ou amino-2, méthylthio-4, butyronitrile), peut être obtenu par réaction de l'ammoniaque sur la cyanhydrine correspondante ou tout autre moyen qui n'apporterait pas de sel dans le milieu.

Enfin, le procédé selon l'invention, de préparation de l'amide sur résine hydroxyde permet, lors du contact avec cette résine, d'éliminer les cyanures/nitriles résiduels qui peuvent exister en sortie de la synthèse de l'aminonitrile et qui sont néfastes dans le cas de l'hydrolyse enzymatique de l'aminoamide en méthioninate d'ammonium et en plus dont la présence est à éviter dans le produit final.

Pour l'hydratation catalysée de l'aminonitrile en aminoamide, les résines sont notamment choisies parmi les résines basiques hydroxydes telles que les résines commerciales vendues sous les marques ROHM & HAAS Ambersep 900 OH, ou FLUKA.

Après un usage prolongé, la résine est éventuellement régénérée par traitement en milieu sodique. La régénération est effectuée de préférence avec une solution de soude selon une concentration pondérale d'au moins 4 % en NaOH et selon une quantité de NaOH de 80 à 150 g/litre de résine.

La présente invention concerne également un procédé industriel de préparation de la méthionine. Selon ce procédé, on fait réagir l'aldéhyde méthylthiopropionique avec l'acide cyanhydrique en présence d'un catalyseur basique ou d'un tampon permettant de maintenir le pH de la solution entre 5,5 et 7,5, on peut citer parmi ces catalyseurs : les amines tertiaires, notamment la triéthylamine ou la pyridine, ou encore le tampon citrate. L'acide cyanhydrique est utilisé soit pur, soit en mélange avec les gaz issus de sa synthèse, tels que l'azote, le dioxyde de carbone, l'oxyde de carbone, l'eau et le méthane, après notamment élimination de l'ammoniac. La réaction est réalisée de préférence à température ambiante et en présence de quantités équimolaires de chacun des réactifs ou de quantités d'acide cyanhydrique légèrement supérieures à la stoechiométrie telle que par exemple selon un excès par rapport à la stoechiométrie d'environ 5 %. La réaction peut avoir lieu en réacteur agité ou en réacteur tubulaire, elle peut aussi avoir lieu en contacteur gaz-liquide pour permettre une absorption réactive ceci de préférence lorsqu'on utilise l'acide cyanhydrique gazeux.

On obtient en fin d'étape de cyanation une solution aqueuse d'hydroxy-2, méthylthio-4, butyronitrile, noté HMTBN (101). Cette solution est mise en contact avec de l'ammoniac (102) ou avec un mélange ammoniac/eau. La quantité molaire d'ammoniac utilisé rapporté à l'HMTBN est comprise avantageusement entre 4 et 7. Les quantités ci-avant précisées étant décrites dans le brevet DE 2645544. L'ammoniac est utilisé pur ou en solution aqueuse. La solution aqueuse d'ammoniac étant de préférence utilisée à une concentration supérieure à 25 % en poids et de préférence à une concentration supérieure à 60 % en poids. On préfère tout particulièrement utiliser l'ammoniac pur. La température de réaction est de préférence située entre 40 et 80°C et tout particulièrement entre 55 et 70°C. On effectue la réaction notamment dans un réacteur (A) agité ou tubulaire avec un écoulement notamment de type piston. En fin de réaction, on obtient une solution aqueuse d'amino-2, méthylthio-4, butyronitrile (106).

En fin de réaction une partie de l'ammoniac excédentaire est éliminée ou séparée par détente de type flash simple ou avec entraînement par un gaz inerte tel que, par exemple, l'azote, la vapeur d'eau, dans une colonne (B). La température du milieu, pendant l'étape de séparation, est inférieure à 60°C et de préférence comprise entre 15 et 50°C. L'ammoniac récupéré (109), en tête de colonne (B), est ensuite absorbé dans un absorbeur (C). La solution d'ammoniaque obtenue (415/422) est ensuite distillée (D). L'ammoniac gazeux obtenu en tête de la colonne de distillation (423) est condensé (E), puis après un éventuel appoint d'ammoniac (426), est de préférence réinjecté sous forme d'un flux (427) dans le réacteur de synthèse de l'aminonitrile. Le flux recyclé d'ammoniac (427) qui est introduit dans le réacteur de synthèse de l'aminonitrile contient de préférence moins de 5 % en poids d'acétone,.

Le pied (424) de colonne de distillation (D), contenant de l'eau, de l'acétone et de l'ammoniac est divisé en deux flux (205) et (410). Le flux minoritaire (205) est renvoyé à la synthèse de l'aminoamide et le flux majoritaire (410) est envoyé à l'absorbeur (C).

L'aminonitrile contenu dans le flux (110) obtenu en pied de colonne (B) et qui contient encore, notamment de 0,1 mole à 1,5 mole d'ammoniac résiduel par mole d'aminonitrile est hydraté en présence d'une cétone (202 et 205), par contact avec une résine échangeuse d'ion de type hydroxyde, dans le réacteur (F).

On préfère utiliser, parmi les cétones, l'acétone. L'hydratation de l'aminonitrile est réalisée en présence de 0,1 à 1, de préférence 0,2 à 0,6 équivalent d'acétone/mole d'AMTBN. L'hydratation est réalisée notamment en présence d'une quantité de résine basique telle que le nombre d'équivalent hydroxyde soit compris notamment entre 0,10 et 1 et de préférence entre 0,15 et 0,5 équivalent/mole d'AMTBN. La température de réaction est de préférence fixée entre 10 et 40°C et encore plus préférentiellement entre 15 et 25°C. La réaction peut être réalisée en continu, discontinu ou semicontinu dans un système de type réacteur agité ou réacteur tubulaire ou enfin dans une colonne contenant la résine basique.

En fin de réaction on obtient une solution aqueuse (208) contenant l'aminoamide de la méthionine, de l'ammoniaque, de l'acétone, de l'eau et divers composés organiques, tels que à titre indicatif mais non limitatif la méthionine ou l'imidazolidinone.

Le mélange (208) obtenu est notamment distillé dans la colonne (G). La tête de colonne (211) est condensée dans le condenseur (H) de façon à récupérer de l'acétone contenant peu d'ammoniac (212). Ce flux est recyclé à la synthèse de l'aminoamide. Le flux d'incondensable (213), contenant essentiellement de l'ammoniac, est envoyé à l'absorbeur (C).

Le mélange aqueux (214) subsistant dans le pied de la colonne de distillation, après élimination de l'acétone, contenant essentiellement l'aminoamide de la méthionine, et les divers composés organiques est alors dilué (303) puis dirigé vers la section hydrolyse de l'aminoamide (I).

Cette hydrolyse peut être réalisée par l'une des 3 voies suivantes:
- par voie chimique en catalyse homogène : hydrolyse à l'ammoniaque. Pour une meilleure mise en oeuvre de l'invention, on utilise de préférence un ratio molaire NH3/AMTBM compris entre 2 et 25mol/mol. On utilise également une concentration en substrat AMTBM compris de préférence entre 0,05 et 1mol/kg. En ce qui concerne les conditions de mise en oeuvre de l'invention on préfère utiliser une température comprise entre 100 et 180°C.
- par voie chimique en catalyse hétérogène . Cette hydrolyse est catalysée notamment par l'un des oxydes suivants: TiO2, TiO2/Al2O3, Nb2O5, Nb2O5-Al2O3, ZnO, ZrO2, et les hétéropolyacides. La réaction est réalisée de préférence à l'ébullition du milieu réactionnel (environ 100°C), sous pression atmosphérique et sous balayage continu d'azote de façon à éliminer l'ammoniac formé. La concentration en substrat AMTBM dans le milieu réactionnel est selon une meilleure manière de mise en oeuvre de l'invention comprise entre 0,1 et 2 mol/kg et le rapport massique catalyseur/substrat entre 0,5 et 1,5.

Cette dernière voie est particulièrement avantageuse par rapport aux procédés décrits dans les brevets JP 03093753, JP 03093754, JP 03093755 et JP 03093756 car:
- elle permet de réaliser la réaction à plus basses température et pression, ce qui permet d'éviter la décomposition thermique des réactifs et produits,
- elle permet de récupérer directement (à chaud) la méthionine acide, sans autre forme d'acidification et donc sans consommation d'acide minéral. La séparation du catalyseur solide et de la méthionine solubilisée à chaud est aisée.
- par voie enzymatique à l'aide d'une amidase. Cette amidase est choisie parmi les amidases de *Rhodococcus* IBN 20 ou de *Brevibacterium* R312. On peut utiliser l'information génétique codant pour l'amidase et l'exprimer dans un micro-organisme hôte. Le micro-organisme hôte est notamment choisi parmi *Escherichia coli* ou un membre du genre *Corynebacterium.* Ce matériel biologique exprimant l'activité amidase est de préférence immobilisé.

Le flux issu de l'hydrolyse de l'amide (305) est alors traité, dans la colonne (J), par entraînement par un gaz inerte (306) tel que choisi par exemple parmi l'azote ou la vapeur d'eau dans une colonne dont la température est régulée par les flux d'entrée des divers gaz. La pression est maintenue dans cette colonne de préférence entre 10⁵ et 2 10⁵ Pa. En tête de colonne (J) on récupère l'ammoniac et une partie de l'eau (308) qui sont cnvoyés dans l'absorbeur (C).

Le pied de la colonne (J) contenant une solution aqueuse de méthionine (311), exempte d'ammoniac, est concentré (K) jusqu'à obtenir une bouillie très concentrée véhiculable jusqu'à la filtration ou tout autre système de séparation (L) où elle peut être séchée complètement jusqu'à l'état pulvérulent.

Les exemples suivants illustrent la mise en oeuvre du procédé selon la présente invention.

### Exemple 1 : Enchaînement HMTBN->AMTBN->AMTBM avec hydratation de l'AMTBN sur résine basique Ambersep 900H de Rohm et Haas et dégazage à 67°C (essai AYP 031)

### Ex.1.1 Préparation de l'aminonitrile de la méthionine

On charge 72g d'une solution commerciale d'ammoniaque Rectapur (30 % p/p) soit 1270 mmoles de NH3, dans un réacteur autoclave inox double enveloppé comportant des chicanes et une agitation magnétique. Après fermeture du réacteur, la solution est chauffée à 67°C, par l'intermédiaire de la double-enveloppe. La pression se stabilise à 4 bars absolus.

Dans une ampoule de coulée métallique, on charge 42 g d'une solution d'HMTBN à 78,5 % p/p dans l'eau, soit 251 mmoles d'HMTBN.

Le contenu de l'ampoule métallique est introduit rapidement dans l'autoclave sous pression, grâce à une contre-pression de 6 bars d'azote.

Le mélange est alors agité pendant 15 minutes à 67°C, sous 6 bars.

Le milieu est ensuite immédiatement dégazé à 67°C.

Après ouverture du réacteur, le milieu d'un poids final de 98,6 g est immédiatement transféré dans un erlen préalablement refroidi à 10°C.

L'expérience est répétée une seconde fois en mettant en jeu les mêmes quantités. Un deuxième milieu réactionnel de 100,4 g est alors récupéré.

Les 2 milieux réactionnels sont alors mélangés et agités à 10°C donnant lieu à une émulsion. L'emulsion est alors passée en ampoule à décanter.

Une phase supérieure aqueuse (jaune claire) de 165,7 g et une phase inférieure organique (jaune foncé) de 31,7 g sont alors obtenues.

L'analyse par chromatographie des 2 phases permet de doser:
- 177 mmoles d'amino-2(méthylthio-4)butyronitrile (AMTBN) dans la phase organique,
- 295 mmoles d'AMTBN dans la phase aqueuse.

Le rendement total de récupération de l'AMTBN par rapport à l'HMTBN introduit est de 94 %.

### Ex.1.2. Préparation de l'aminoamide de la méthionine

Dans un réacteur en verre agité de 11, muni d'une turbine Rushton et de chicanes, on charge 60 g de résine basique Ambersep 900H de Rohm et Haas à 1,5 milliéquivalent OH/g (soit 90 mmoles d'équivalent OH, et donc 0,19 mole de base/mole d'AMTBN) et 65 g d'eau.

La suspension est agitée et maintenue à 12°C

La totalité des 2 phases obtenues dans l'exemple 1.1. (soit 472 mmoles d'AMTBN) est engagée sur cette suspension, ainsi que 13,9 g d'acétone (soit 239,6 mmoles et donc 0,51 mole d'acétone/mole d'AMTBN), sous agitation à 500 T/min. La température du mélange est portée et maintenue à 20°C.

Après 2 h d'agitation, une analyse chromatographique montre que la totalité de l'AMTBN a réagi.

Le milieu est laissé au total 5 h sous agitation, puis filtré. Le filtrat est évaporé sous vide à 45°C.

Le liquide visqueux de couleur jaune obtenu se solidifie à température ambiante en 30 min. Un poids total de 61,9 g de ce solide est alors récupéré.

Une analyse RMN du solide obtenue montre une pureté molaire de 96 % en aminoamide, 2 % en imidazolidinone, 0,5 % en méthionine et 1,5 % de divers organiques.

Une analyse d'eau par la méthode Karl-Fisher donne une teneur pondérale d'eau de 1,4 % sur le solide récupéré.

Une analyse chromatographique du même solide donne des teneurs pondérales de 93 % en aminoamide, 3 % en imidazolidinone et 0,5 % en méthionine et des divers organiques.

Les rendements de récupération des produits suivants par rapport à l'AMTBN engagé lors de la deuxième étape sont de 82,3 % en aminoamide, 2,1 % en imidazolidinone et 0,4 % en méthionine.

### Exemple 2 : Enchaînement HMTBN->AMTBN->AMTBM avec hydratation de l'AMTBN sur résine basique Ambersep 900H de Rohm et Haas et dégazage à 33°C (essai AYP 035)

### Ex.2.1 Préparation de l'aminonitrile de la méthionine

On charge 58,6g d'une solution d'HMTBN à 78,8 % p/p dans l'eau, soit 352 mmoles d'HMTBN dans un réacteur autoclave inox double enveloppe comportant des chicanes et une agitation magnétique. Après fermeture du réacteur, la solution est chauffée à 51,5°C, par l'intermédiaire de la double-enveloppe et d'un bain thermostaté.

Dans une ampoule de coulée métallique, maintenue dans de la carboglace et préalablement mise sous vide, on transfert 30,8 g d'ammoniac gazeux provenant d'une bouteille (soit 1812 mmoles de NH3).

Le contenu de l'ampoule métallique est introduit rapidement dans l'autoclave sous pression, grâce à une contre-pression de 10 bars d'azote.

La température du mélange agité se stabilise et est maintenue à 67°C par le bain thermostaté. Le mélange est agité pendant 10 minutes sous une pression de 12 bars absolus.

Le réacteur fermé est ensuite refroidi à 33°C en 30 minutes, la pression diminuant à 7 bars absolus, puis dégazé.

Après ouverture du réacteur, le milieu d'un poids de 74,3 g est immédiatement refroidi à 10°C et passé en ampoule à décanter.

Une phase supérieure aqueuse (jaune claire) de 19,79 g et une phase inférieure organique (jaune foncé) de 52,09 g sont alors obtenues.

L'analyse par chromatographie des 2 phases permet de doser:
- 319,5 mmoles d'AMTBN dans la phase organique,
- 32,6 mmoles d'AMTBN dans la phase aqueuse.

Le rendement total de récupération de l'AMTBN par rapport à l'HMTBN introduit est de 100%.

### Ex.2.2. Préparation de l'aminoamide de la méthionine

Dans un réacteur en verre agité de 11, muni d'une turbine Rushton et de chicanes, on charge 41,9 g de résine basique Ambersep 900H de Rohm et Haas à 1,5 méquivalent OH/g (soit 62,9 mmoles d'équivalent OH) et 88,2 g d'eau.

La suspension est agitée et maintenue à 15°C.

62,5g du mélange des 2 phases obtenues dans l'exemple 2.1.(soit 306 mmoles d'AMTBN) sont engagées sur cette suspension, ainsi que 9,2 g d'acétone (soit 158,6 mmoles), sous agitation à 360 T/min. La température du mélange est portée et maintenue pendant 3 h à 20°C.

Le milieu est ensuite filtré. Le filtrat est évaporé sous vide (1 mm de Hg) à 30°C.

Le liquide visqueux de couleur jaune obtenu se solidifie à température ambiante. Un poids total de 43,8 g de ce solide est alors récupéré.

Une analyse chromatographique du même solide donne des teneurs pondérales de 78 % en aminoamide, 11 % en imidazolidinone et 3 % en méthionine et des divers organiques.

Les rendements de récupération des produits suivants par rapport à l'AMTBN engagé à la deuxième étape sont de 75,3 % en aminoamide, 10,6 % en imidazolidinone et 2,9 % en méthionine.

### Exemple 3 : Hydrolyse de l'AMTBM à l'ammoniaque fessai AYP 036)

On charge 52 g d'une solution commerciale d'ammoniaque Rectapur (30 % p/p) soit 918 mmoles de NH3, dans un réacteur autoclave inox double enveloppé comportant des chicanes et une agitation magnétique. Après fermeture du réacteur, la solution est chauffée à 134°C, par l'intermédiaire de la double-enveloppe, sous agitation.

Dans une ampoule de coulée métallique, on charge 27,2 g d'une solution d'AMTBM à 1,4 mol/kg, réalisée en mélangeant 7,3 g de l'aminoamide préparé à l'exemple 2.2 (soit 38,4mmoles d'AMTBM) et 19,9 g d'eau.

Le contenu de l'ampoule métallique est introduit rapidement dans l'autoclave sous pression, grâce à une contre-pression de 20 bars d'azote. Le mélange ainsi effectué est à 0,49 mol/kg en AMTBM. Il est alors agité à 150°C, sous pression.

Après 1 h 30 de réaction, un rendement en méthionine de 53 % (compté par rapport à l'AMTBM et l'imidazolidinone de départ) est obtenu.

Après 7 h de réaction, un rendement en méthionine de 81 % est ainsi obtenu.

### Exemple 4 : Hydrolyse de l'AMTBM à l'ammoniaque (essai AYP 038)

Dans cet exemple, le même mode opératoire que dans l'exemple 3 précédent, est suivi, en utilisant:
- 88,8 g d'un mélange constitué de 22,8 g d'une solution commerciale d'ammoniaque Rectapur (30 % p/p) soit 402 mmoles de NH3 et de 66,0 g d'eau,
- 23,7 g d'une solution d'AMTBM à 0,7 mol/kg, réalisée en mélangeant 3,2 g de l'aminoamide préparé à l'exemple 2.2 (soit 16,8 mmoles d'AMTBM) et 20,5 g d'eau.

Le mélange obtenu est à 0,15 mol/kg en AMTBM.

Après 1 h 30 de réaction à 150°C, un rendement en méthionine de 72 % (compté par rapport à l'AMTBM et l'imidazolidinone de départ) est obtenu.

Après 7 h de réaction, un rendement quantitatif en méthionine est ainsi obtenu.

### Exemple 5 : Hydrolyse de l'AMTBM sur catalyseur TiO2 (essai ASE 065)

On charge, dans un réacteur en verre, double-enveloppé, 66,2 g d'eau et 7,98 g de catalyseur TiO2 préalablement broyé. La seule sortie du réacteur est reliée à une série de barboteurs contenant de l'acide sulfurique dilué (2 % p/p).

La suspension, contenue dans le réacteur, est mise en agitation et chauffée à 94°C, sous circulation d'azote.

Une solution aqueuse d'AMTBM à 1,35 mol/kg, réalisée en mélangeant 10 g d'aminoamide brut de l'exemple 2.2 (soit 52,6 mmoles d'AMTBM) et 29,02 g d'eau, est introduite dans le réacteur, au moyen d'une pompe.

Le solution ainsi obtenu dans le réacteur est à 0,5 mol/kg d'AMTBM.

Après 30 minutes de réaction, la conversion de l'aminoamide est totale. Le rendement en méthionine obtenu est de 85 %.

### Exemple 6 : Hydrolyse de l'AMTBM sur catalyseur TiO2/Al2O3 (essai ASE 064)

Dans cet exemple, le même mode opératoire que dans l'exemple 5 précédent, est suivi, en utilisant:
- 8,0 g de catalyseur TiO2/Al2O3 à 3 % en poids de Ti,
- 10,94 g d'amide brut de l'exemple 2.2 (soit 57,6 mmoles d'AMTBM) et un total de 92,7 g d'eau.

Le solution ainsi obtenu dans le réacteur est à 0,56 mol/kg d'AMTBM.

Après 7 heures de réaction, la conversion de l'aminoamide est de 52 % environ. Le rendement en méthionine obtenu est de 30 % environ.

### Exemple 7: Hydrolyse enzymatique de l'AMTBM par Corynebacterium Glutamicum pYG822 (essais GF571 et 590).

La souche *corynebacterium glutamicum* pYG822, décrite dans le brevet FR N°9014853 (28.11.90), exprime l'amidase de *Rhodococcus* IBN 20. Le *corynebacterium* est cultivée 24 heures à 30°C dans 1 litre de milieu contenant 10 g de tryptone, 5 g d'extrait de levure, 5 g de NaCl et 20 mg de Kanamycine. Le culot cellulaire est récupéré par centrifugation. On charge dans un réacteur le culot cellulaire, 0,1 litre de tampon phosphate 100 mM pH 7,0 et 2,2 g d'AMTBM provenant de l'exemple 2.2. Après réaction de 6 heures à 35°C la conversion de l'aminoamide est de 100%. Le rendement en méthionine est de 100 %.

### Exemple 8 : Hydrolyse enzymatique de l'AMTBM par Escherichia coli pXL1751 (essai GF 584).

La souche *Escherichia coli* pXL1751, décrite dans le brevet EP N° 433 117(91-179908B) exprime l'amidase de *Brevibacterium* R312. L'*E*. *coli* est cultivée 24 heures à 37°C dans 1 litre de milieu M9 (DIFCO) contenant 4 g de Casaminoacids, 5 g d'extrait de levure et 100 mg d'ampicilline. Le culot cellulaire est récupéré par centrifugation. On charge dans un réacteur le culot cellulaire, 0,1 litre de tampon phosphate 100 mM pH 7,0 et 2,2 g d'AMTBM provenant de l'exemple 2.2. Après réaction de 6 heures à 35°C la conversion de l'aminoamide est de 100 %. Le rendement en méthionine est de 100 %.

### Contre-exemple 1 : Hydrolyse de l'AMTBM sur Al2O3 (essai ASE 063)

Dans cet exemple, le même mode opératoire que dans l'exemple 5 précédent, est suivi, en utilisant:
- 8,4 g de catalyseur Al2O3 en billes,
- 10,95 g d'amide brut de l'exemple 2.2 (soit 57,6 mmoles d'AMTBM) et un total de 108 g d'eau.

Le solution ainsi obtenu dans le réacteur est à 0,48 mol/kg d'AMTBM.

Après 7 heures de réaction, la conversion de l'aminoamide et le rendement en méthionine sont pratiquement nuls.

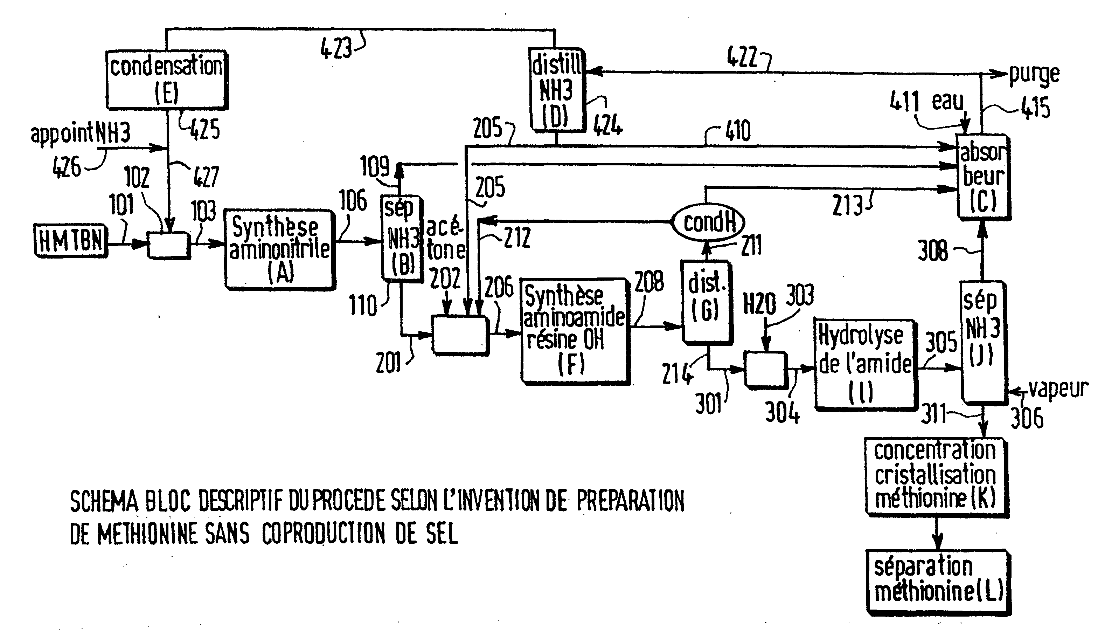

## Revendications

1. Procédé de préparation de l'aminoamide de la méthionine (ou amino-2, méthylthio-4, butyramide), **caractérisé en ce que** on hydrate l'aminonitrile de la méthionine en présence d'un catalyseur de type cétone et d'une résine basique de type hydroxyde.

2. Procédé selon la revendication 1 **caractérisé en ce que** la cétone utilisée est l'acétone.

3. Procédé selon les revendications 1 et 2 **caractérisé en ce que** la quantité molaire d'acétone utilisée par rapport à l'aminonitrile est comprise entre 0.2 et 0.6.

4. Procédé selon la revendication 1 **caractérisé en ce que** la quantité de résine basique utilisée par rapport à l'aminonitrile est comprise entre 0.10 et 1 équivalent hydroxyde et de préférence entre 0.15 et 0.5 équivalent.

5. Procédé selon la revendication 1 **caractérisé en ce que** la température réactionnelle est comprise entre 10 et 40°C et de préférence entre 15 et 25°C.

6. Procédé selon la revendication 1 **caractérisé en ce que** la réaction est réalisée en continu, discontinu ou semicontinu.

7. Procédé de préparation de la méthionine **caractérisé en ce que** dans une première étape l'aminonitrile de la méthionine est hydraté en aminoamide de la méthionine en présence d'un catalyseur de type cétone et d'une résine basique de type OH, dans une deuxième étape l'aminoamide de la méthionine est hydrolysé en méthioninate d'ammonium, par voie chimique en catalyse homogène, par voie chimique en catalyse hétérogène ou par voie enzymatique, dans une troisième étape la méthionine est récupérée, à partir du méthioninate d'ammonium après élimination de l'ammoniac.

8. Procédé selon la revendication 7 **caractérisé en ce que** l'hydrolyse de l'aminoamide de la méthionine est réalisée par voie chimique en catalyse homogène en présence d'ammoniaque.

9. Procédé selon la revendication 7 **caractérisé en ce que** l'hydrolyse de l'aminoamide de la méthionine est réalisée par voie chimique en catalyse hétérogène en présence d'oxydes métalliques ou d'hétéropolyacides.

10. Procédé selon la revendication 9 **caractérisé en ce que** les oxydes métalliques sont choisis parmi le dioxyde de titane, l'oxyde de zinc, le mélange dioxyde de titane pentoxyde de nobium, le dioxyde de zirconium et le pentoxyde de nobium.

11. Procédé selon la revendication 7 **caractérisé en ce que** l'hydrolyse de l'aminoamide de la méthionine est réalisée par voie enzymatique en catalyse hétérogène en présence d'une amidase.

12. Procédé selon la revendication 11 **caractérisé en ce que** l'amidase est issue des micro-organismes *Rhodococcus* ou *Brevibacterium.*

13. Procédé selon les revendications 11 et 12 **caractérisé en ce que** l'on utilise l'information génétique codant pour une amidase que l'on exprime dans un micro-organisme hôte.

14. Procédé selon la revendication 11 à 13 **caractérisé en ce que** le micro-organisme hôte est choisi parmi *Escherichia coli* ou un membre du genre *corynebacterium.*

15. Procédé selon les revendications 11 à 14 **caractérisé en ce que** le matériel biologique exprimant l'activité amidase est immobilisé.

## Claims

1. Process for preparing methionine aminoamide (or 2-amino-4-methylthiobutyramide), **characterized in that** methionine aminonitrile is hydrated in the presence of a catalyst of ketone type and of a basic resin of hydroxide type.

2. Process according to Claim 1, **characterized in that** the ketone used is acetone.

3. Process according to Claims 1 and 2, **characterized in that** the molar amount of acetone used with respect to the aminonitrile is between 0.2 and 0.6.

4. Process according to Claim 1, **characterized in that** the amount of basic resin used with respect to the aminonitrile is between 0.10 and 1 hydroxide equivalent, and preferably between 0.15 and 0.5 equivalent.

5. Process according to Claim 1, **characterized in that** the reaction temperature is between 10 and 40°C, preferably between 15 and 25°C.

6. Process according to Claim 1, **characterized in that** the reaction is carried out continuously, discontinuously or semicontinuously.

7. Process for preparing methionine, **characterized in that**, in a first step, methionine aminonitrile is hydrated to methionine aminoamide in the presence of a catalyst of ketone type and of a basic resin of OH type, in a second step, the methionine aminoamide is hydrolysed to ammonium methioninate, via the chemical pathway by homogeneous catalysis, via the chemical pathway by heterogeneous catalysis or via the enzymatic pathway and, in a third step, the methionine is recovered, from the ammonium methioninate after elimination of the ammonia.

8. Process according to Claim 7, **characterized in that** the methionine aminoamide hydrolysis is carried out via the chemical pathway by homogeneous catalysis in the presence of aqueous ammonia.

9. Process according to Claim 7, **characterized in that** the methionine aminoamide hydrolysis is carried out via the chemical pathway by heterogeneous catalysis in the presence of metal oxides or of heteropolyacids.

10. Process according to Claim 9, **characterized in that** the metal oxides are chosen from titanium dioxide, zinc oxide, the titanium dioxidenobium pentoxide mixture, zirconium dioxide and nobium pentoxide.

11. Process according to Claim 7, **characterized in that** the methionine aminoamide hydrolysis is carried out via the enzymatic pathway by heterogeneous catalysis in the presence of an amidase.

12. Process according to Claim 11, **characterized in that** the amidase is derived from the *Rhodococcus* or *Brevibacterium* microorganisms.

13. Process according to Claims 11 and 12, **characterized in that** use is made of the genetic information encoding an amidase, which is expressed in a host microorganism.

14. Process according to Claim 11 to 13, **characterized in that** the host microorganism is chosen from *Escherichia coli* or a member of the *corynebacterium* genus.

15. Process according to Claims 11 to 14, **characterized in that** the biological material expressing the amidase activity is immobilized.

## Patentansprüche

1. verfahren zum Herstellen des Aminoamids von Methionin (oder 2-Amino-4-methylthio-butyramid), **dadurch gekennzeichnet, dass** das Aminonitril von Methionin in Gegenwart eines Katalysators vom Keton-Typ und eines basischen Harzes vom Hydroxid-Typ hydratisiert wird.

2. Verfahren nach Anspruch 1, **dadurch gekennzeichnet, dass** das eingesetzte Keton Aceton ist.

3. Verfahren nach den Ansprüchen 1 und 2, **dadurch gekennzeichnet, dass** die molare Menge des eingesetzten Acetons bezüglich des Aminonitrils zwischen 0,2 und 0,6 liegt.

4. verfahren nach Anspruch 1, **dadurch gekennzeichnet, dass** die Menge des eingesetzten basischen Harzes bezüglich des Aminonitrils zwischen 0,10 und 1 Hydroxidäquivalent, und vorzugsweise zwischen 0,15 und 0,5 Äquivalent, liegt.

5. Verfahren nach Anspruch 1, **dadurch gekennzeichnet, dass** die Reaktionstemperatur zwischen 10 und 40°C, und vorzugsweise zwischen 15 und 25°C, liegt.

6. Verfahren nach Anspruch 1, **dadurch gekennzeichnet, dass** die Reaktion kontinuierlich, diskontinuierlich oder semikontinuierlich durchgeführt wird.

7. Verfahren zum Herstellen von Methionin, **dadurch gekennzeichnet, dass** in einem ersten Schritt das Aminonitril von Methionin in das Aminoamid von Methionin in Gegenwart eines Katalysators vom Keton-Typ und eines basischen Harzes vom OH-Typ hydratisiert wird, dass in einem zweiten Schritt das Aminoamid des Methionins in das Ammoniummethionat hydrolysiert wird, und zwar auf chemischem Wege mit homogener Katalyse, auf chemischem Wege mit heterogener Katalyse oder auf enzymatischem Wege, und dass in einem dritten Schritt das Methionin aus dem Ammoniummethionat nach Entfernen des Ammoniaks gewonnen wird.

8. Verfahren nach Anspruch 7, **dadurch gekennzeichnet, dass** die Hydrolyse des Aminoamids von Methionin auf chemischem Wege mit homogener Katalyse in der Gegenwart von Ammoniak durchgeführt wird.

9. verfahren nach Anspruch 7, **dadurch gekennzeichnet, dass** die Hydrolyse des Aminoamids von Methionin auf chemischem Wege mit heterogener Katalyse in der Gegenwart von Metalloxiden oder Heteropolysäuren durchgeführt wird.

10. Verfahren nach Anspruch 9, **dadurch gekennzeichnet, dass** die Metalloxide ausgewählt sind aus Titandioxid, zinkoxid, der Mischung von Titandioxid/Niobpentoxid, zirconiumdioxid und Niobpentoxid.

11. Verfahren nach Anspruch 7, **dadurch gekennzeichnet, dass** die Hydrolyse des Aminoamids von Methionin auf enzymatischem Wege mit heterogener Katalyse in der Gegenwart einer Amidase durchgeführt wird.

12. Verfahren nach Anspruch 11, **dadurch gekennzeichnet, dass** die Amidase von den Mikroorganismen *Rhodococcus* oder *Brevibacterium* stammt.

13. Verfahren nach den Ansprüchen 11 und 12, **dadurch gekennzeichnet, dass** die genetische Information herangezogen wird, die für eine Amidase kodiert, welche in einem Wirts-Mikroorganismus exprimiert wird.

14. Verfahren nach den Ansprüchen 11 bis 13, **dadurch gekennzeichnet, dass** der Wirts-Mikroorganismus ausgewählt ist aus *Escherichia coli* oder einem Mitglied der Gattung *Corynebaterium*.

15. Verfahren nach den Ansprüchen 11 bis 14, **dadurch gekennzeichnet, dass** das biologische Material, das die Amidaseaktivität exprimiert, immobilisiert ist.
